# EUROPEAN PATENT APPLICATION

(11) **EP 2 341 043 A1**
(43) Date of publication of application: **06.07.2011**
(21) Application number: 09811564.5
(22) Date of filing: 28.08.2009
(51) Int. Cl.: C07C 211/54, C09K 11/06, H01L 51/50

(54) **NOVEL 1,3,5-TRIS(DIARYLAMINO)BENZENE AND USE THEREOF**

(30) Priority: 04.09.2008 JP 2008226619
(71) Applicant: Bando Chemical Industries, Ltd., Kobe-shi, Hyogo 650-0047 (JP)
(72) Inventor: OHTA, Masashi, Kobe-shi Hyogo 6500047 (JP); AKASHI, Nobutaka, Kobe-shi Hyogo 6500047 (JP)
(74) Representative: Benson, John Everett
(86) International application number: PCT/JP2009/065461
(87) International publication number: WO 2010/027039

(57) **Abstract**

The invention provides a 1,3,5-tris(diarylamino)benzene represented by the general formula (I) 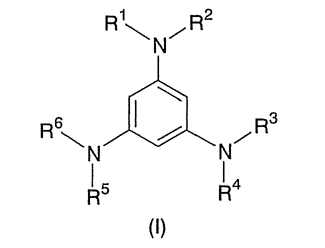 in which R¹ to R⁶ are each independently a group which is represented by the general formula (II) 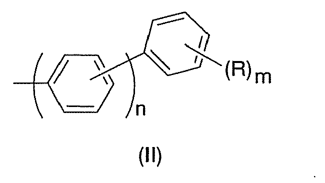 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that at least one of R¹ to R⁶ is independently a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

## Description

### Field of the Invention

The invention relates to a novel 1,3,5-tris(diarylamino)benzene and use thereof. More particularly, the invention relates to a novel 1,3,5-tris(diarylamino)benzene which is useful as a heat resistant and amorphous organic electronic material because it has at least one aryl group having two or more alkyl or cycloalkyl groups as substituents at least on one of the three amino group nitrogen atoms it has, and has a high crystallization temperature and a high amorphousity as well as a high solubility to solvents. The invention further relates to use of such a 1,3,5-tris(diarylamino)benzene, in particular, as an organic electronic functional material such as a hole transporting agent in an organic electroluminescence element and so on.

### Background Art

In recent years, a variety of electronic devices such as light-emitting elements or semiconductors in which an organic compound which has photoelectric function as well as reversible oxidation-reduction characteristics and can form amorphous film by itself is used as an organic electronic functional material, for example, as a hole transporting agent, have attracted considerable attention. It is known that such amorphous film of organic substances can be formed by preparing a coating composition comprised of a binder resin such as polycarbonate resin and the organic compound dissolved in a suitable organic solvent and then by coating the composition on a substrate and drying the composition (patent literature 1). It is also known that in the case of a polynuclear aromatic tertiary amine called a "star-burst" compound, the compound is vacuum evaporated onto a substrate to form thin film directly (patent literature 2).

According to a method using a binder resin among the methods mentioned above, the organic compound is diluted with the binder resin in the resulting thin film and influenced by the binder resin so that the organic compound cannot exhibit sufficiently the functions as an organic electronic functional material that it originally has. In addition, if the organic compound forms thin film that is stable at normal temperature with the aid of a binder resin, the organic compound has a low glass transition temperature so that the film is poor in heat resistance and has a problem in stability and life.

Under these circumstances, polynuclear aromatic tertiary amine compounds called the "star-burst" molecules are attracting considerable attention as organic electronic functional materials since they are capable of forming stable thin film by vacuum evaporation. The star-burst molecules are divided into three groups based on their molecular structures: compounds having a triphenylamine structure (triphenylamines), compounds having a triaminobenzene structure (triaminobenzenes) and compounds having a triphenylbenzene structure (triphenylbenzenes).

A variety of "star-burst" molecules have been proposed, and for example, 1,3,5-tris(N-p-methylphenyl)-N-(1-naphthyl)aminobenzene (p-MTPNAB) and 1,3,5-tris(N-p-methylphenyl)-N-(4-biphenylyl)-amino)benzene (p-MTPBAB) have been proposed as examples of the triaminobenzenes (patent literature 3). Both the above-mentioned two triaminobenzenes are reversible in oxidation-reduction processes and have relatively high glass transition temperatures, i.e., 87°C and 98°C, respectively. However, there is still a fear that they have not enough stability in performance and durability for use as organic electronic functional material.

Such being the case, 1,3,5-tris(N-(4'-methyl-4-biphenylyl)-N-(4-methylphenyl)amino)benzene (referred to as the compound (1) hereinafter) represented by the following formula (1) 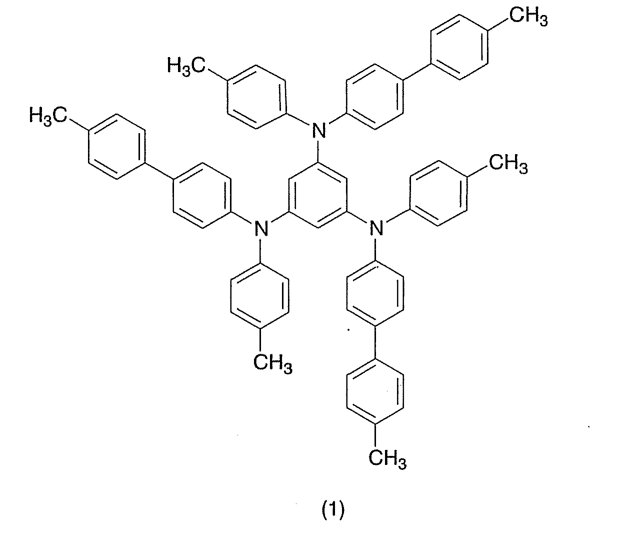 has recently been proposed (patent literature 4).

Although this 1,3,5-tris(diarylamino)benzene has not only a better stability in repeated oxidation-reduction processes but also a much higher glass transition temperature than the known 1,3,5-tris-(diarylamino)benzenes, it has a relatively low crystallization temperature, and therefore there is a fear that the amorphous film thereof crystallizes when it is put under high temperature atmosphere. Therefore, it has a problem that it is poor in heat stability. If amorphous film crystallizes in this way, the performance of organic electronic functional material formed of the 1,3,5-tris(diarylamino)benzene deteriorates remarkably. That is, an electronic devices in which such a 1,3,5-tris(diarylamino)benzene is used is not sufficient in heat resistance.
Patent literature 1: JP 11-174707A
Patent literature 2: JP 08-291115A
Patent literature 3: JP 2004-155754A
Patent literature 4: JP 2005-190993A

### Summary of the Invention

### Technical Problem

The invention has been completed to solve the problems involved in the known 1,3,5-tris(diarylamino)benzenes as mentioned above. Therefore, it is an object of the invention to provide a novel 1,3,5-tris(diarylamino)benzene which has a high crystallization temperature and hardly crystallizes if it is put under high temperature environment while it retains excellent properties as an organic electronic functional material, and hence it is superior in amorphousity and is capable of providing a highly heat resistant organic electronic functional material, and in addition, on account of high solubility to solvents, it is easy to synthesize and easy to treat when it is to be used.

### Solution to Problem

The invention provides a 1,3,5-tris(diarylamino)benzene represented by the general formula (I) 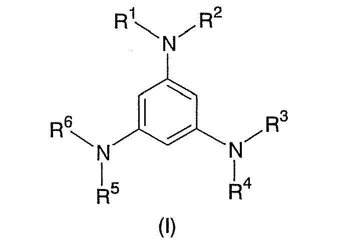 in which R¹ to R⁶ are each independently a group which is represented by the general formula (II) 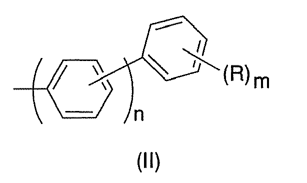 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that at least one of R¹ to R⁶ is independently a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

In particular, the invention provides, as a first of preferred embodiments, a 1,3,5-tris(diarylamino)benzene represented by the general formula (III) 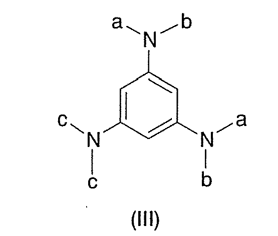 in which a, b and c are each independently a group represented by the general formula (II) 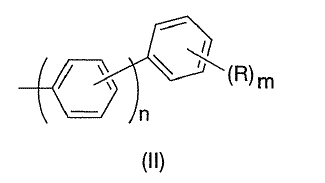 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

The invention further provides, as a second of preferred embodiments, a 1,3,5-tris(diarylamino)benzene represented by the general formula (IV) 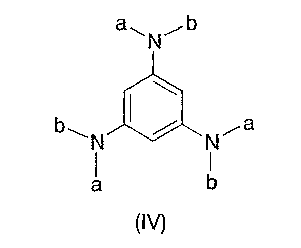 wherein a and b are each independently a group represented by the general formula (II) 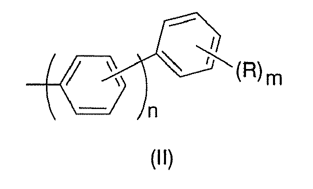 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

The invention still further provides an organic electronic functional material comprising the 1,3,5-tris(diarylamino)benzene represented by the general formula (I).

The organic electronic functional material comprising the 1,3,5-tris(diarylamino)benzene of the invention is preferably used as a hole injecting agent or a hole transporting agent. Therefore, the invention provides as a preferred embodiment an organic electroluminescence element comprising a hole injecting layer and/or a hole transporting layer comprising the 1,3,5-tris(diarylamino)benzene as a hole injecting agent and/or a hole transporting agent, respectively.

### Advantageous Effects of the Invention

The 1,3,5-tris(diarylamino)benzenes of the invention have a high crystallization temperature and a high amorphousity as well as a high solubility to solvents because they have such a structural feature that they have at least one aryl group having two or more alkyl or cycloalkyl groups as substituents at least on one of the three amino group nitrogen atoms they have, and hence they are useful as a heat resistant and amorphous organic electronic functional material. Furthermore, the 1,3,5-tris(diarylamino)benzenes of the invention are easy to synthesize and easy to treat when they are to be used as an organic electronic functional material.

### Brief Description of Drawings

FIG. 1 is a sectional view showing an example of an organic electroluminescence element;
FIG. 2 is an infrared absorption spectrum of the compound (2) of the invention;
FIG. 3 is a DSC chart of the compound (2) of the invention;
FIG. 4 is a TG/DTA chart of the compound (2) of the invention;
FIG. 5 is a CV chart of the compound (2) of the invention;
FIG. 6 is an infrared absorption spectrum of the compound (4) of the invention;
FIG. 7 is a DSC chart of the compound (4) of the invention;
FIG. 8 is a TG/DTA chart of the compound (4) of the invention;
FIG. 9 is a CV chart of the compound (4) of the invention; and
FIG. 10 is a graph showing the relative luminance of an organic electroluminescence element of the invention and an organic electroluminescence element as a comparative example when they are heated from 40°C to 200°C letting the luminance at 40°C be 100%.

### Reference Signs List

- 1 ...: Transparent substrate
- 2 ...: Anodes (Transparent electrode)
- 3a ...: Hole injecting layer
- 3b ...: Hole transporting layer
- 4 ...: Light emitting layer
- 5 ...: Cathode
- 6 ...: Power supply

### Description of Embodiments

The 1,3,5-tris(diarylamino)benzene of the invention is represented by the general formula (I) 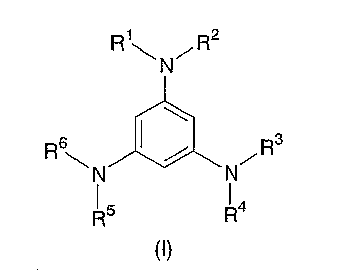 in which R¹ to R⁶ are each independently a group which is represented by the general formula (II) 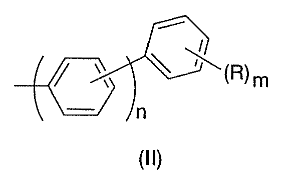 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that at least one of R¹ to R⁶ is independently a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

In the 1,3,5-tris(diarylamino)benzene represented by the general formula (I), when R is an alkyl group in the general formula (II), the alkyl group is exemplified by a methyl, an ethyl, a propyl, a butyl, a pentyl or a hexyl group, and the alkyl group having three carbons or more may be linear or branched. When R is a cycloalkyl group in the general formula (II), the cycloalkyl group is exemplified by a cyclopentyl or a cyclohexyl group.

Herein the invention, the group which is represented by the general formula (II) and constitutes at least one of R¹ to R⁶, and in which R is an alkyl group of 1 to 6 carbon atoms or a cycloalkyl group of 5 or 6 carbon atoms, m is 2, 3, 4 or 5, and n is 0, 1 or 2, is referred to as the substituted aryl group.

According to the invention, it is particularly preferred that m in the substituted aryl group is 2, and in such a case, it is preferred that at least one of two R's is situated at an ortho position of the terminal phenyl group. The reason therefor is as follows. Because of steric effect of R caused when R is situated at an ortho position of the terminal phenyl group of the substituted aryl group in this manner, the terminal phenyl group is distorted from planarity that involves the nitrogen atom to which the terminal phenyl group is bonded to destroy the planarity (when n is 0), or alternatively, the terminal phenyl group is distorted from planarity that involves the phenyl group adjacent to the terminal phenyl group to destroy the planarity (when n is 1 or 2). As results, the 1,3,5-tris(diarylamino)benzene of the invention has an enhanced amorphousity. In the above-mentioned, when n is 0, the ortho position of the terminal phenyl group, that is, of the substituted aryl group, is a position adjacent to a position at which the phenyl group is bonded to an amino nitrogen atom, while when n is 1 or 2, the ortho position of the terminal phenyl group of the substituted aryl group is a position adjacent to a position at which the terminal phenyl group is bonded to a phenyl group adjacent thereto.

Further according to the invention, it is particularly preferred that one of the two R's is situated at an ortho position while the other at the para position. It is also preferred that both the two R's are alkyl groups, and particularly, methyl groups. In the group represented by the general formula (II), the para position of the terminal phenyl group is an active site in reactions. Thus, when the para position of the terminal phenyl group is substituted by R, the 1,3,5-tris(diarylamino)benzene of the invention is enhanced in stability, and hence the stability of 1,3,5-tris(diarylamino)benzene of the invention as an organic electronic functional material is enhanced.

Among the 1,3,5-tris(diarylamino)benzenes represented by the general formula (I), the particularly preferred first of embodiments of the 1,3,5-tris(diarylamino)benzenes of the invention is represented by the general formula (III) 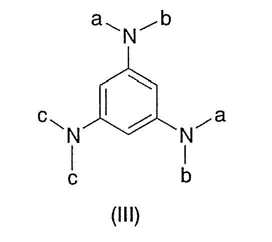 in which a, b and c are each independently a group represented by the general formula (II) 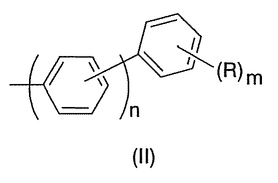 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

According to the invention, in the 1,3,5-tris(diarylamino)-benzenes represented by the general formula (III), a is preferably a group represented by the general formula (IIa) 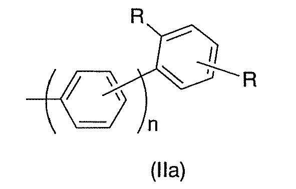 in which the two R's are each independently an alkyl group of 1 to 6 carbon atoms or a cycloalkyl group of 5 or 6 carbon atoms, and n is 0, 1 or 2, and b and c are each preferably independently a group represented by the general formula (IIb) 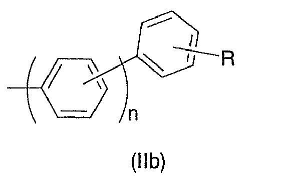 wherein R is independently a hydrogen atom, an alkyl group of 1 to 6 carbon atoms or a cycloalkyl group of 5 or 6 carbon atoms, and n is 0, 1 or 2.

Most preferably, in the 1,3,5-tris(diarylamino)benzenes represented by the general formula (III), a is a group represented by the above-mentioned general formula (IIa), and one of the two R's is situated at an ortho position and the other ay the para position by the same reason as before, and the two R's are alkyl groups, and in particular, methyl groups.

Therefore, in such a preferred first of the embodiments of the 1,3,5-tris(diarylamino)benzenes of the invention, the groups b and c are, for example, a phenyl group, a biphenylyl group or a terphenylyl group, and the phenyl group, and the terminal phenyl group when the groups b and c are a biphenylyl group or a terphenylyl group, may have an alkyl group or a cycloalkyl group as the substituent R.

Examples of such preferred first of the embodiments of 1,3,5-tris(diarylamino)benzenes of the invention include N,N'-bis(2,4-dimethylphenyl)-N,N'-bis(4-terphenylyl)-N",N"-bis(4-methylphenyl)-1,3,5-triaminobenzene represented by the formula (2) 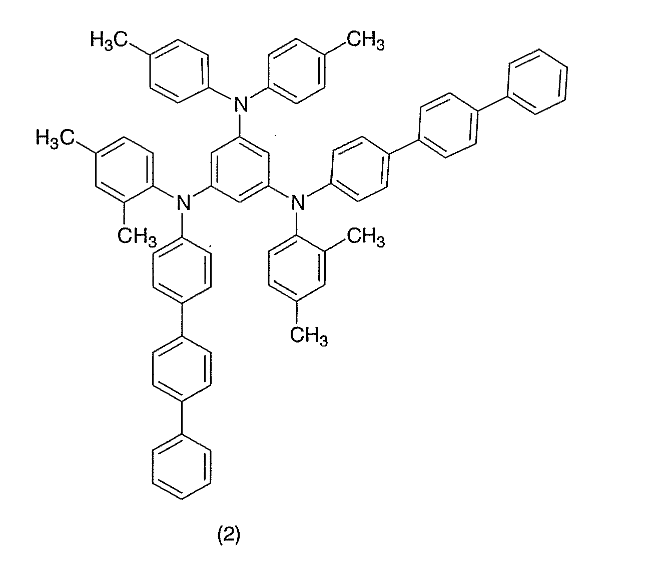 (referred to as the compound (2) hereafter), and N,N'-bis(2,4-dimethylphenyl)-N,N'-bis(4-terphenylyl)-N",N"-diphenyl-1,3,5-triaminobenzene represented by the formula (3) 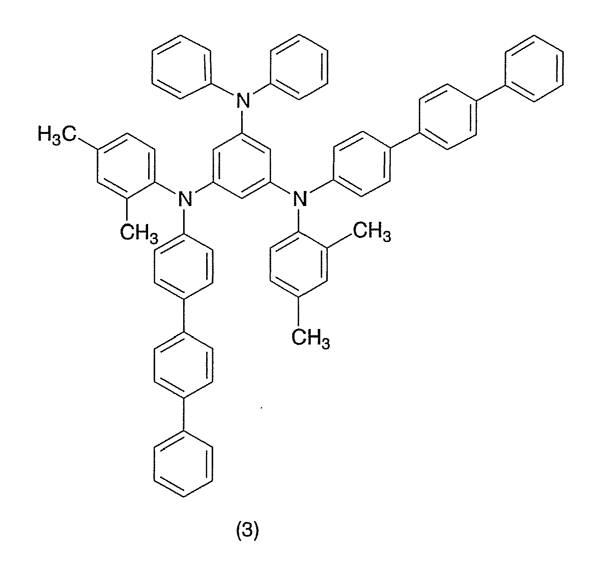 (referred to as the compound (3) hereafter).

In turn, among the 1,3,5-tris(diarylamino)benzenes represented by the general formula (I), the particularly preferred second of embodiments of the 1,3,5-tris(diarylamino)benzenes of the invention is represented by the general formula (IV) 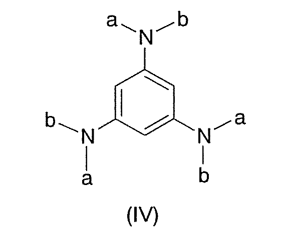 in which a and b are each independently a group represented by the general formula (II) 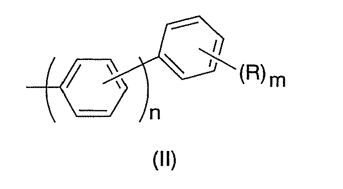 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

In particular, among the 1,3,5-tris(diarylamino)benzenes represented by the general formula (IV), it is preferred that a is the group represented by the general formula (IIa) mentioned above, and b is independently the group represented by the general formula (IIb) mentioned above.

Among the 1,3,5-tris(diarylamino)benzenes represented by the general formula (IV), it is most preferred that a is the group represented by the general formula (IIa) mentioned above, and one of the two R's is situated at an ortho position and the other at the para position as mentioned hereinbefore, and the two R's are alkyl groups, in particular, methyl groups.

Therefore, also in such a preferred second of embodiments of the 1,3,5-tris(diarylamino)benzenes of the invention, the group b is, for example, a phenyl group, a biphenylyl group or a terphenylyl group, and the phenyl group, and the terminal phenyl group when b is a biphenylyl group or a terphenylyl group, may have an alkyl group or a cycloalkyl group as the substituent R.

Examples of such preferred second of embodiments of the 1,3,5-tris(diarylamino)benzenes of the invention include N,N',N"-tris-(2,4-dimethylphenyl)-N,N',N"-tris(4-terphenylyl)-1,3,5-triaminobenzene represented by the formula (4) 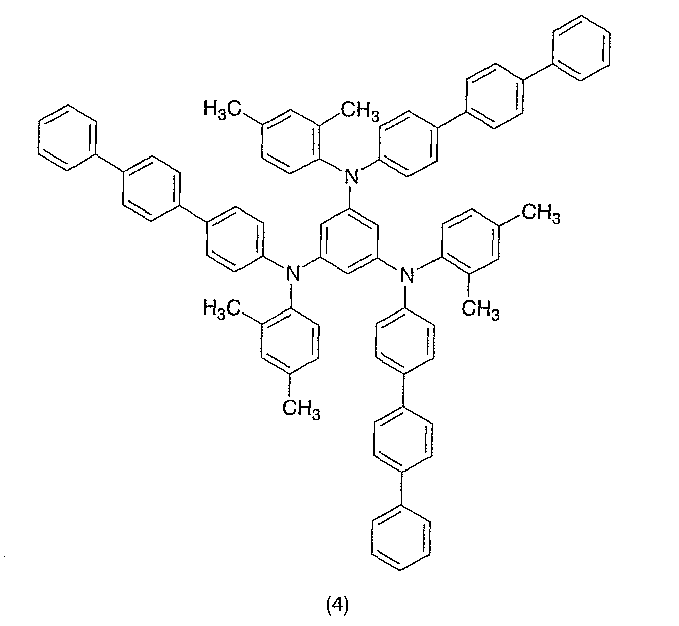 (referred to as the compound (4) hereafter), and N,N',N"-tris-(2,4-dimethylphenyl)-N,N',N"-tris(4-terphenylyl)-1,3,5-triaminobenzene represented by the formula (5) 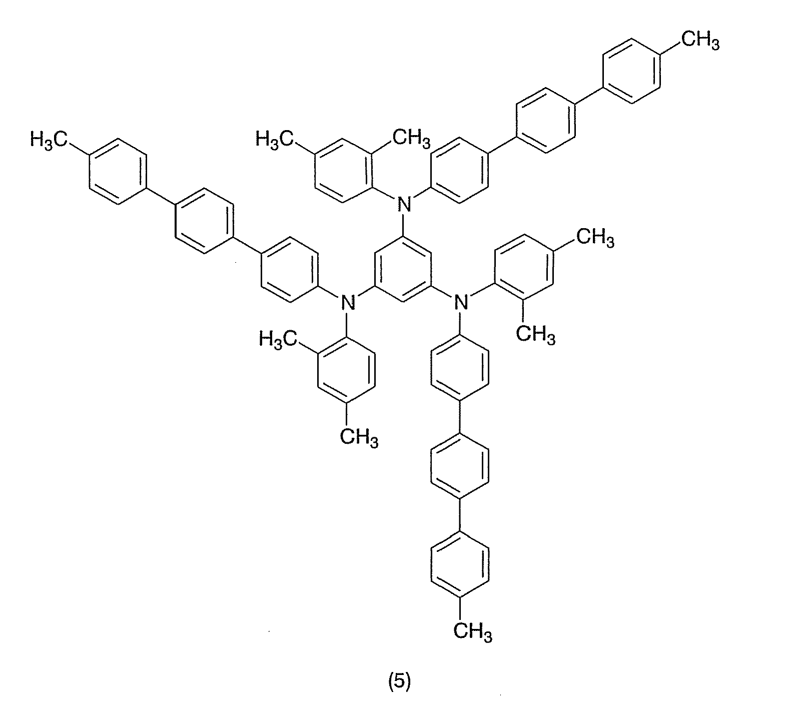 (referred to as the compound (5) hereafter).

The compound (2) can be obtained in a manner as follows, for example. One molar part of N,N-bis(4-methylphenyl)amino-3,5-dichlorobenzene is reacted with two molar parts of 2,4-dimethylphenyl-4-terphenylylamine using tri-t-butylphosphine and a palladium catalyst such as palladium acetate in the presence of a base in a reaction solvent under an inert atmosphere. An alkali metal alkoxide such as sodium t-butoxide, and a carbonate or a hydrogen carbonate of alkali metal such as sodium carbonate and sodium hydrogen carbonate are used as the base. A hydrocarbon solvent such as xylene, decalin, mesitylene, and heptane is preferably used as the reaction solvent. The reaction temperature is usually in the range of 100-130°C, and the reaction time is usually in the range of 5-30 hours. After completion of reaction, the obtained reaction product is dissolved in an organic solvent, and the catalyst is remove by filtration. The reaction product is then separated and purified by column chromatography using an appropriate eluate. If necessary, recrystallization is carried out from an appropriate organic solvent to provide purified product.

The compound (4) can also be likewise obtained by reacting one molar part of 1,3,5-tribromobenzene with three molar parts of 2,4-dimethylphenyl-4-terphenylylamine.

The 1,3,5-tris(diarylamino)benzene of the invention has a high crystallization temperature and a high amorphousity as well as a high solubility to solvents because of the structural feature that it has at least one aryl group having two or more alkyl groups and/or cycloalkyl groups at least on one of the three amino group nitrogen atoms it has. Accordingly, the 1,3,5-tris(diarylamino)benzene of the invention is useful as a heat resistant and amorphous organic electronic functional materials for use in, for example, organic electroluminescence elements.

That is, the 1,3,5-tris(diarylamino)benzene of the invention can form film by vacuum evaporation, and in addition, as the film thus obtained is amorphous film with no anisotropy because the film shows no clear peaks by analysis using X-ray diffractometry. Therefore, the 1,3,5-tris(diarylamino)benzene of the invention can form stable and heat resistant amorphous film at an ordinary temperature or higher by itself, namely, without aid of a binder resin. Moreover, the 1,3,5-tris-(diarylamino)benzene of the invention has such a high solubility to organic solvents that it is easy to synthesize and it is convenient in handling.

The 1,3,5-tris(diarylamino)benzene of the invention is suitable for use as an organic electronic functional material in applications where heat resistance is required in various electronic devices. Among various electronic devices, an organic electroluminescence element in particular can be driven by direct current at a low electric voltage with high efficiency to emit light at a high luminance, as well as it can be made thin. Accordingly, in recent years, the investigation to put the organic electroluminescence element to practical use as display devices as well as backlights or illumination devices is pushed forward. As mentioned above, the 1,3,5-tris(diarylamino)benzene of the invention can be preferably used as various kinds of organic electronic functional materials, for example, a hole injecting agent, a hole transporting agent, a host agent in an emitting layer, a hole blocking agent in a hole blocking layer, an electron blocking agent in an electron blocking layer, etc.

By way of examples, a durable and high luminance organic electroluminescence element which can be driven at a low voltage can be obtained either by placing a hole injecting layer including an organic electronic functional material comprising the 1,3,5-tris(diarylamino)-benzene of the invention as a hole injecting agent between an anode and a hole transporting layer, or by placing a hole transporting layer including an organic electronic functional material comprising the 1,3,5-tris-(diarylamino)benzene of the invention as a hole transporting agent between a hole injecting layer and an emitting layer, or by placing a hole injecting/transporting layer including an organic electronic functional material comprising the 1,3,5-tris(diarylamino)benzene of the invention as a hole injecting/transporting agent between an anode and an emitting layer,

However, it goes without saying that the 1,3,5-tris(diarylamino)-benzene of the invention can be used to form thin film as a hole injecting and/or transporting layer using a binder resin.

Thus, the 1,3,5-tris(diarylamino)benzene of the invention can be used as a hole injecting agent to form a hole injecting layer in an organic electroluminescence element, and accordingly, the 1,3,5-tris(diarylamino)benzene of the invention can be used as a hole transporting agent to form a hole transporting layer as the hole injecting agent has the same function as the hole transporting agent. When a hole injecting layer and a hole transporting layer are formed as a single layer in an organic electroluminescence element, the 1,3,5-tris(diarylamino)benzene of the invention can be used as a hole injecting/transporting agent.

As mentioned above, the 1,3,5-tris(diarylamino)benzene of the invention can be used as a hole injecting and/or transporting agent, and such a hole injecting and/or transporting agent can be used as a hole injecting and/or transporting layer in an organic electroluminescence element.

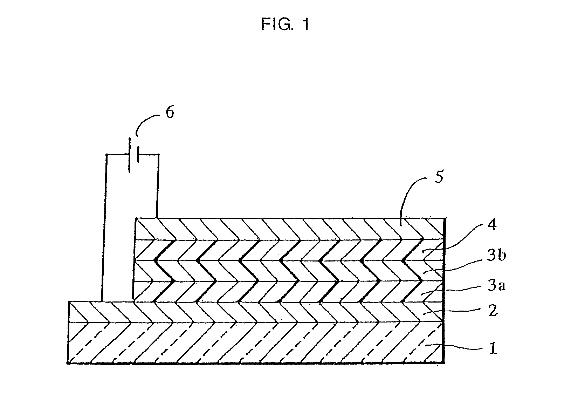

As a preferred example of an electroluminescence element according to the invention is shown in Fig. 1, it comprises a transparent substrate 1 made of glass, for example, having an anode 2 made of a transparent electrode such as an ITO membrane (indium oxide-tin oxide membrane) laminated thereon, and a hole injecting layer 3a, a hole transporting layer 3b, an emitting layer 4 and a cathode 5 made of a metal or a compound thereof laminated on the anode in this order. The anode and the cathode are connected with an external power source 6. In such an organic electroluminescence element as mentioned above, holes are readily injected from the anode into the emitting layer through the hole injecting layer and the hole transporting layer so that the organic electroluminescence element can be driven at a low electric voltage. Electrons are injected into the emitting layer from the cathode. The electrons injected from the cathode and the holes injected from the anode recombine in the emitting layer, and light is emitted and radiated outside through the transparent electrode (anode) and the transparent substrate.

According to the invention, in some cases, an electron transporting layer may be laminated between the emitting layer and the cathode, and a blocking layer may be formed in order to prevent excessive holes from passing through the emitting layer towards the cathode. The organic electroluminescence element is not specifically limited in layer structure.

The preferred electroluminescence element of the invention comprises a hole injecting and/or transporting layer which comprise the 1,3,5-tris(diarylamino)benzene of the invention. The 1,3,5-tris-(diarylamino)benzene of the invention is capable of forming amorphous film by itself, and accordingly, a hole injecting layer can be formed by vacuum evaporating the 1,3,5-tris(diarylamino)benzene of the invention onto the transparent electrode using a suitable vacuum evaporation device. The thickness of hole injecting layer is usually in the range of 5 nm to 200 nm, preferably in the range of 10 nm to 80 nm. A hole transporting layer can be also formed by vacuum evaporating the 1,3,5-tris(diarylamino)benzene of the invention onto a hole injecting layer appropriately formed. The thickness of hole transporting layer is usually in the range of 5 nm to 200 nm, preferably in the range of 10 nm to 80 nm. Needless to say, a single layer of hole injecting/transporting layer comprising the 1,3,5-tris(diarylamino)benzene of the invention can be formed on a transparent anode.

However, the 1,3,5-tris(diarylamino)benzene of the invention may be dissolved in a suitable organic solvent, and if needed, together with an appropriate binder resin, to prepare a coating composition, and the coating composition may be applied onto an anode with an appropriate coating means such as a spin coat method, and then dried, thereby preparing a hole injecting and/or transporting layer. Also in this case, the thickness of the layer is the same as mentioned above.

When a hole injecting and/or transporting layer comprising the 1,3,5-tris(diarylamino)benzene of the invention as a hole injecting and/or transporting agent is used in the organic electroluminescence element of the invention, the layers except the hole injecting and/or transporting layer, that is, a transparent substrate, an ordinary hole injecting and/or transporting layer to be combined with the hole injecting and/or transporting layer according to the invention, an anode, an emitting layer, an electron transporting layer and a cathode, may be made of any conventionally known materials. For example, an anode or a transparent electrode may be made of indium oxide-tin oxide (ITO), and a cathode may be made of a metal such as aluminum, magnesium, indium or silver, or an alloy of these metals, such as Al-Mg alloy, Ag-Mg alloy, or lithium fluoride. A transparent substrate is usually made of glass.

As the ordinary hole transporting agent, any known low molecular weight organic compounds, such as α-NPD (4,4'-bis(N-(1-naphthyl)-N-phenylamino)biphenyl) and TPD (4,4'-bis(3-methylphenyl)-N-phenylamino)biphenyl are used. As the ordinary hole injecting agent, for example, copper phthalocyanine is used. The thickness of the layer is usually in the range of 10-200 nm. Tris(8-quinolinol)aluminum (Alq₃), for example, is used for an emitting layer. The thickness of the emitting layer is usually in the range of 10-200 nm. When the organic electroluminescence element contains an electron transporting layer, the thickness thereof is usually in the range of 10-200 nm.

The 1,3,5-tris(diarylamino)benzene of the invention is not specifically limited in its uses, and it can be preferably used as a hole injecting agent, a hole transporting agent, a host agent in an emitting layer, a hole blocking agent, an electron blocking agent and so on in an organic electroluminescence element, and in addition is can be used also as an organic semiconductor in a solar battery, a charge transporting material in an electrophotography device among others.

### Examples

The invention will now be explained with reference to examples below, but the invention is not limited by the examples.

### Example 1

### (Synthesis of the compound (2))

0.027 g of palladium acetate, 4.05 g of N,N-bis(4-(methylphenyl)-amino-3,5-dichlorobenzene (a), 9.1 g of 2,4-dimethylphenyl-4-terphenylylamine (b), 7.6 g of sodium t-butoxide, and 0.048 g of tri-t-butylphosphine were added in this order to 120 mL of xylene in a 500 mL capacity separable flask, and were heated to 105°C with stirring, followed by six hour-reaction at the temperature with stirring.

After 200mL of ethanol was added to the resulting reaction mixture to cease the reaction, the resulting solid was collected by filtration, and purified by column chromatography. The solid thus obtained was further recrystallized from 100 mL of toluene, thereby 9.0 g of white solid was obtained in a yield of 78%.

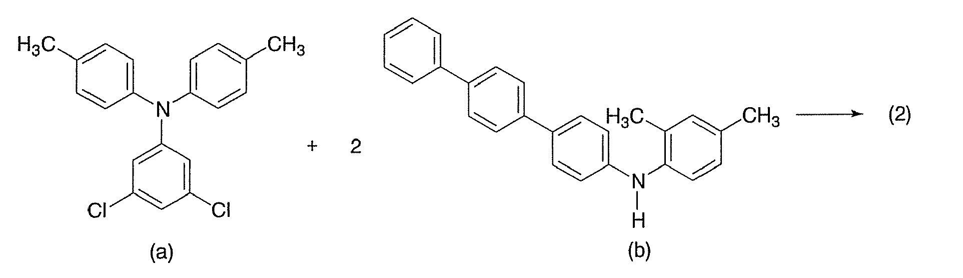

| | | | |
|---|---|---|---|
| Elemental analysis (%) as C₇₂H₆₁N₃: | | | |

| | C | H | N |
|---|---|---|---|
| Calculated | 89.31 | 6.35 | 4.34 |
| Measured | 89.46 | 6.32 | 4.22 |

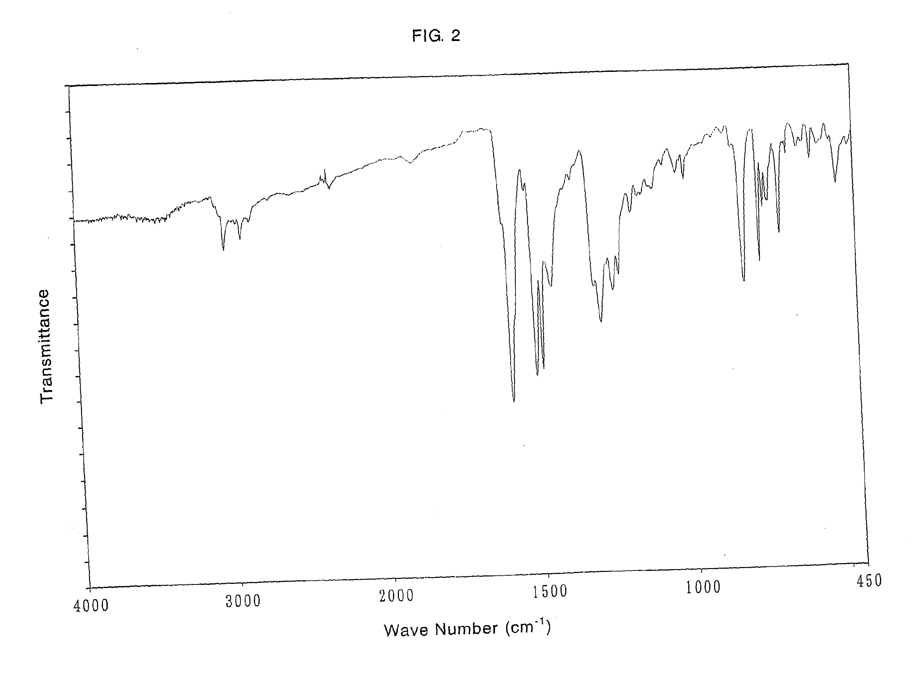

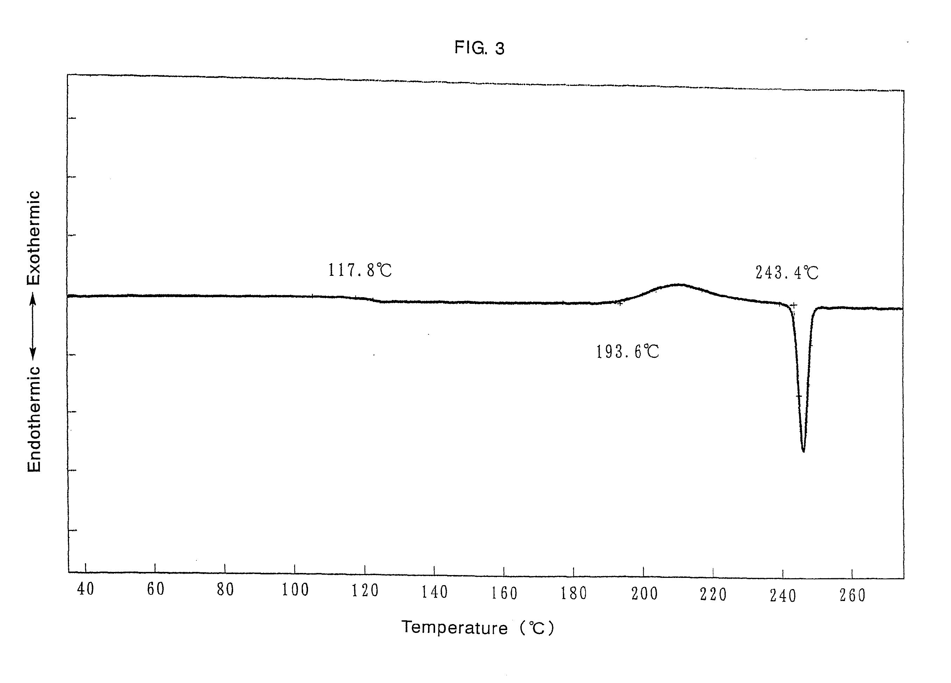

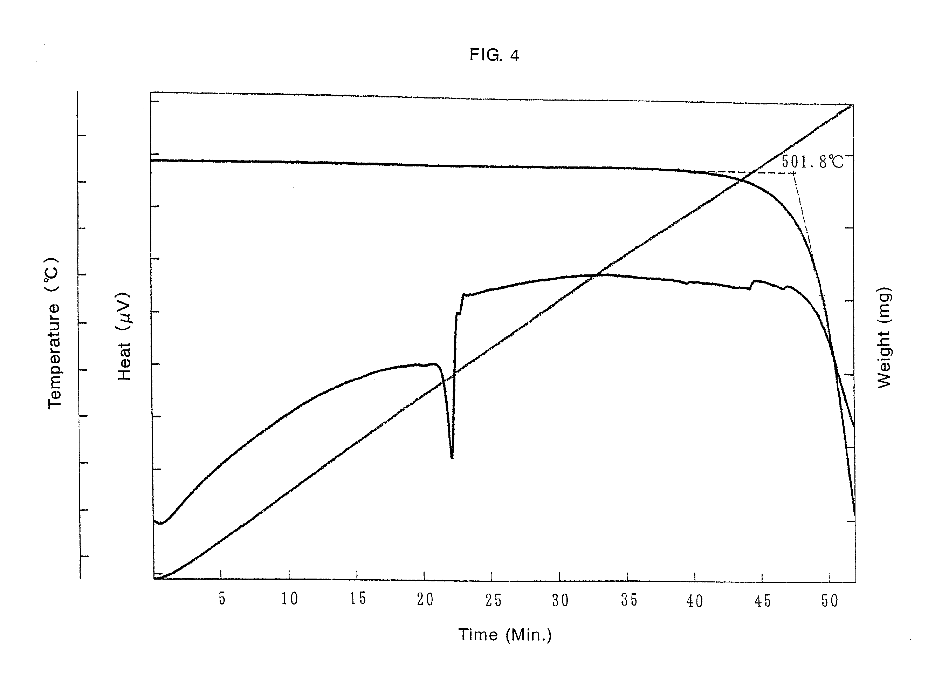

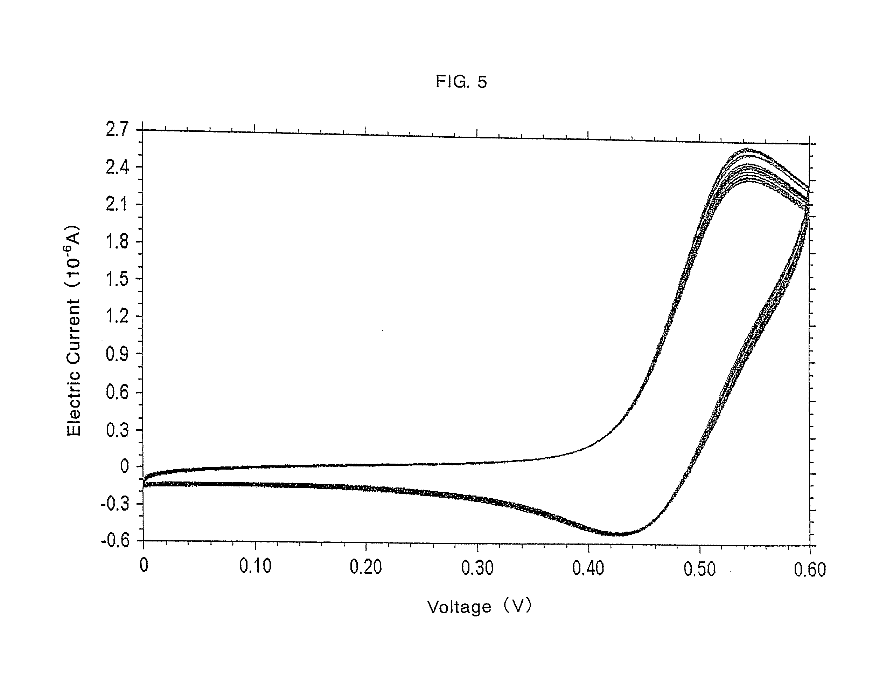

Mass analysis
M⁺=969
IR spectrum:
The IR spectrum is shown in Fig. 2.
Differential scanning calorimetry (DSC):
As the DSC chart is shown in Fig. 3, the compound was found to have a glass transition temperature (Tg) of 117°C, a crystallization temperature (Tc) of 193°C, and a melting point (Tm) of 243°C.
Thermal properties:
The TG/DTA chart is shown in Fig. 4.
Cyclic voltammetry (CV):
As the CV chart is shown in Fig. 5, the compound was found to have an oxidation potential of 0.49 V (vs. Ag/Ag⁺).

### Example 2

### (Synthesis of the compound (4))

0.043 g of palladium acetate, 6.14 g of 1,3,5-tribromobenzene (c), 22.5 g of 2,4-dimethylphenyl-4-terphenylylamine (b), 11.2 g of sodium t-butoxide, and 0.079 g of tri-t-butylphosphine were added in this order to 200 mL of xylene in a 500 mL capacity separable flask, and were heated to 105°C with stirring, followed by four hour-reaction at the temperature with stirring.

After 200 mL of ethanol was added to the resulting reaction mixture to cease the reaction, the resulting solid was collected by filtration, and purified by column chromatography, thereby 13.5 g of yellowish white solid was obtained in a yield of 62%.

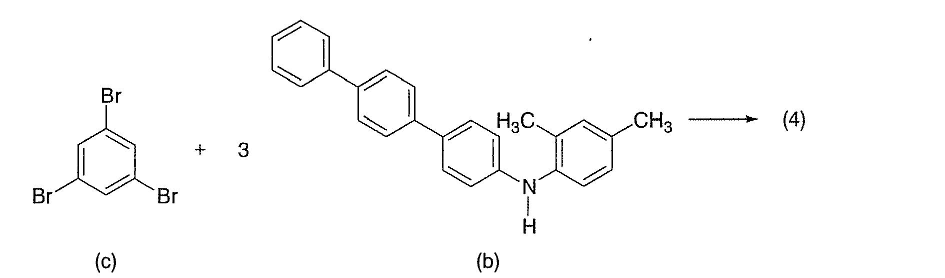

| Elemental analysis (%) as C₈₄H₆₉N₃: | | | |
|---|---|---|---|
| | C | H | N |
| Calculated | 90.04 | 6.21 | 3.75 |
| Measured | 89.92 | 6.40 | 3.68 |

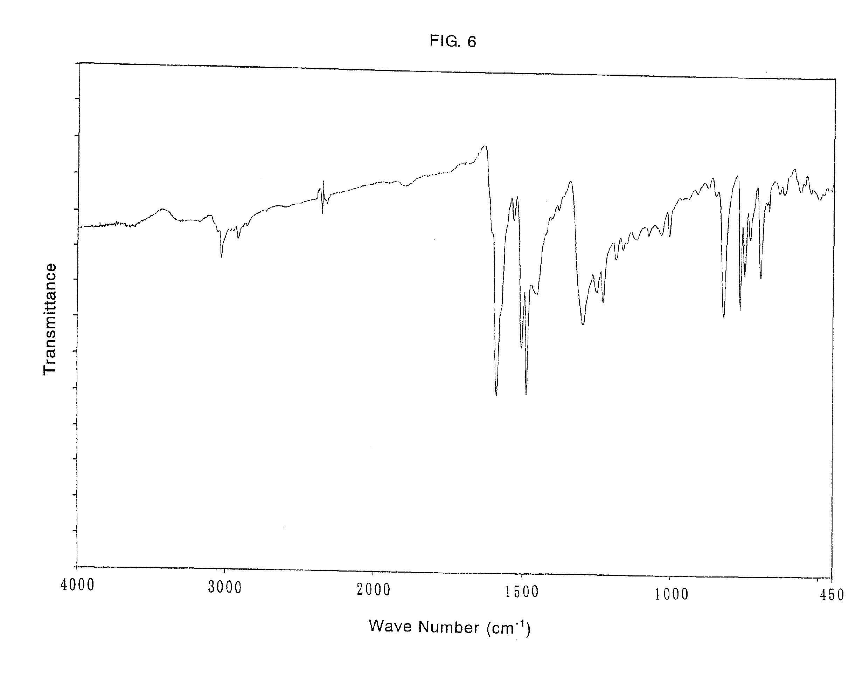

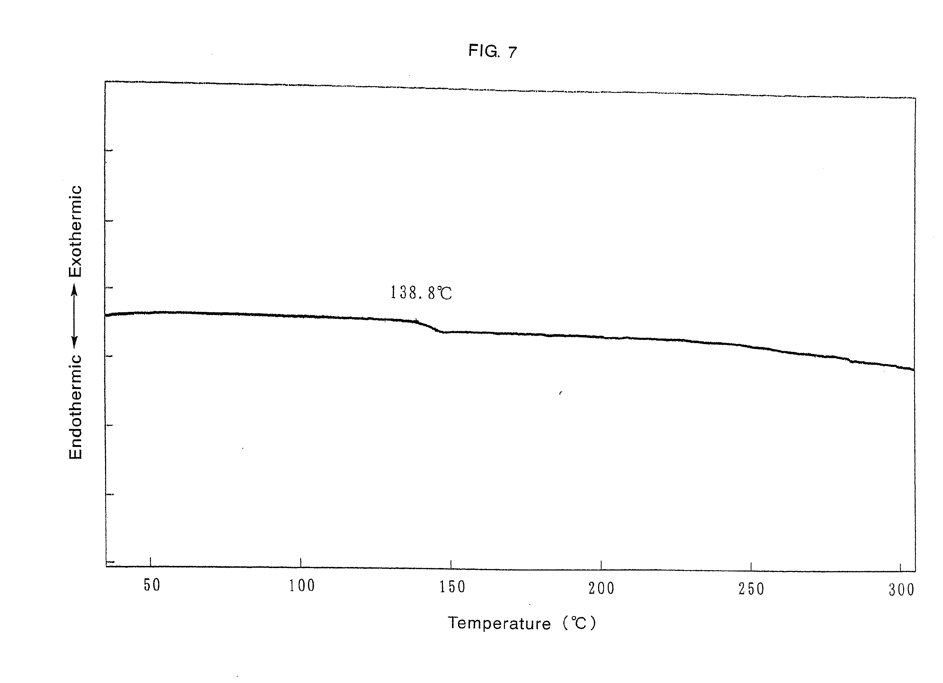

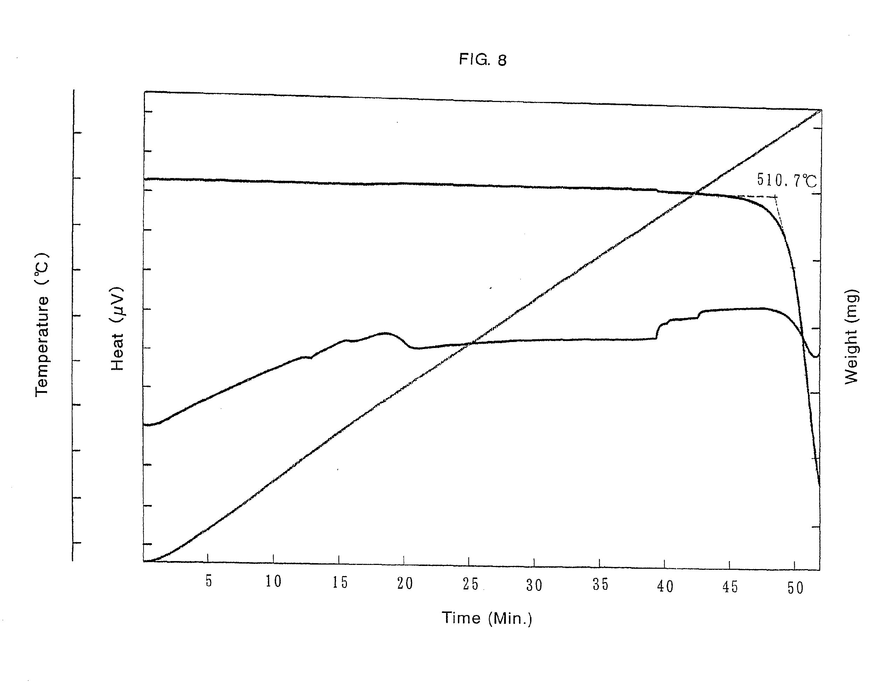

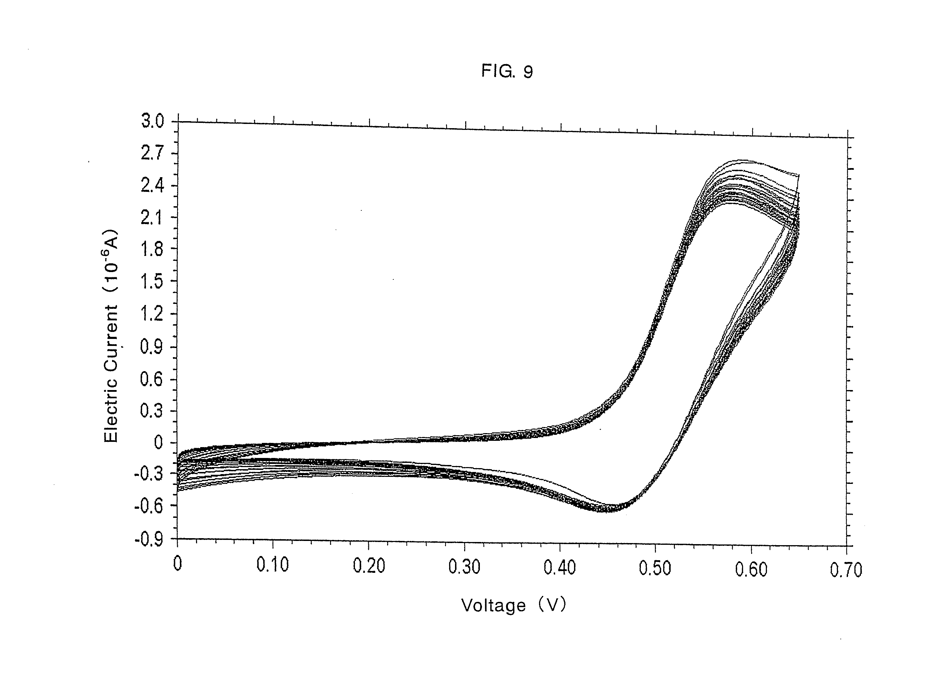

Mass analysis
M⁺=1221
IR spectrum:
The IR spectrum is shown in Fig. 6.
Differential scanning calorimetry (DSC):
As the DSC chart is shown in Fig. 7, the compound was found to have a glass transition temperature (Tg) of 138°C.
Thermal properties:
The TG/DTA chart is shown in Fig. 8.
Cyclic voltammetry (CV):
As the CV chart is shown in Fig. 9, the compound was found to have an oxidation potential of 0.51 V (vs. Ag/Ag⁺).

The glass transition temperatures, crystallization temperatures and solubilities to hot toluene of the compound (2), the compound (4) and the compound (1) are shown in Table 1. The solubility to hot toluene is measured as follows.

A sample in an amount about 1.5 to 2 times as much as the sample was soluble was added to 100 mL of heat toluene and refluxed with stirring for one hour. Thereafter, the sample that had not dissolved in the heat toluene was collected by filtration, and the weight thereof was subtracted from the weight of the sample which was first added to the hot toluene, thereby the amount of the sample dissolved in the hot toluene was measured. Based on the dissolved amount thus measured, the amount of hot toluene required for dissolving 1 g of the sample was calculated as the solubility of the sample to hot toluene.

**TABLE 1**

| | Glass Transition Temperature (Tg) (°C) | Crystallization Tempearture (Tc) (°C) | Solubility (mL/g) |
|---|---|---|---|
| Compound (2) | 117 | 193 | 10 |
| Compound (4) | 138 | not found | 5 |
| Compound (1) | 103 | 139 | 200 |

The 1,3,5-tris(diarylamino)benzenes of the invention, that is, the compounds (2) and (4) have glass transition temperatures higher than the conventionally known compound (1), and the compounds (2) has a crystallization temperature higher than the conventionally known compound (1), while the compound (4) has no crystallization temperature. Accordingly, the 1,3,5-tris(diarylamino)benzenes of the invention are excellent in amorphousity and can be suitably used as a heat resistant organic electronic functional material. For instance, the use of 1,3,5-tris(diarylamino)benzenes of the invention provides an organic electroluminescence element excellent in heat resistance.

Furthermore, both the compounds (2) and (4) are much more soluble to hot toluene than the conventionally known compound (1), and thus they are convenient in preparation and use thereof.

### Example 3

A sheet of plate glass having an ITO coating on one face (available from Sanyo Vacuum K.K.) was subjected to ultrasonic cleaning using acetone and then steam cleaning using methanol, followed by irradiation with ultraviolet rays by using a low-pressure mercury lamp for 10 minutes. Immediately after the irradiation, copper phthalocyanine (CuPC) was vacuum evaporated on the ITO-coated glass to form a hole injecting layer 50 nm thick, and then the compound (2) was vacuum evaporated on the hole injecting layer to form a hole transporting layer 10 nm thick. Subsequently, an emitting layer 75 nm thick was formed of tris(8-quinolinol)aluminum (Alq₃) on the hole transporting layer, and then a lithium fluoride layer 0.5 nm thick and an aluminum layer 100 nm thick were layered in this order on the emitting layer to form a cathode, thereby an organic electroluminescence element was obtained.

### Example 4

In place of the compound (2), the compound (4) was used to form a hole injecting layer 50 nm thick, and otherwise in the same manner as in Example 3, an organic electroluminescence element was obtained.

### Comparative Example 1

In place of the compound (2), α-NPD was used to form a hole injecting layer 50 nm thick, and otherwise in the same manner as in Example 3, an organic electroluminescence element was obtained.

The current efficiency of luminance (cd/A) and the energy efficiency of luminance (lm/W) obtained when the organic electroluminescence element was driven at a current density of 25 mA/cm² are shown in Table 2 together with the luminance half-life period (h) as defined by driving time until the luminance came to half the initial luminance of 1000 cd/m² and the maximum luminance (cd/m²).

**TABLE 2**

| | Current Efficiency of Luminance (@25mA/cm²) (cd/A) | Energy Efficiency of Emission (@25mA/cm²) (lm/W) | Luminance Half-Life Period (@1000cd/m²) (h) | Maximum Luminance (cd/m²) |
|---|---|---|---|---|
| Example 3 | 5.7 | 3.4 | 13966 | 33827 |
| Example 4 | 5.5 | 3.1 | 15329 | 32741 |
| Comparative 1 | 4.2 | 2.4 | 8128 | 29136 |

The organic electroluminescence element of the invention is superior in all of current efficiency of luminance, energy efficiency of emission, luminance half-life period and maximum luminance to the organic electroluminescence element of comparative example.

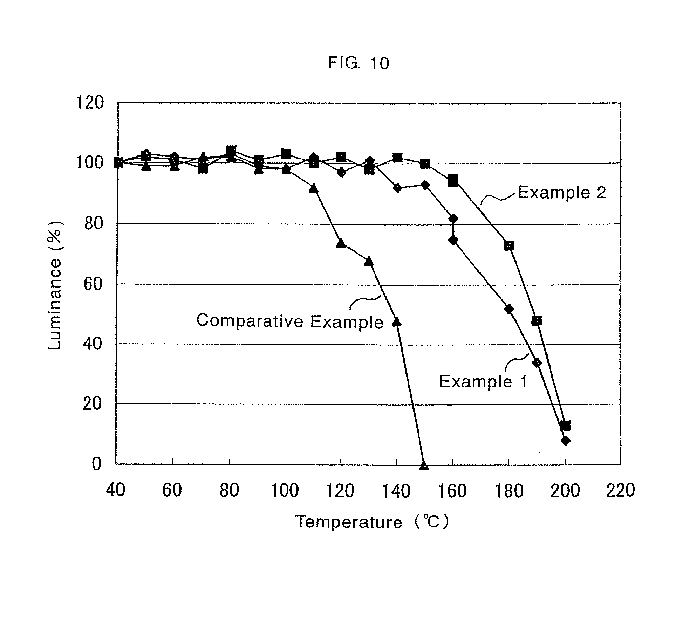

Furthermore, the relative luminance of emission of the organic electroluminescence elements Examples 3 and 4, and Comparative Example from 40°C to 200°C is shown in Fig. 10 letting the luminance at 40°C be 100%. The organic electroluminescence element of the invention is superior in heat resistance to the organic electroluminescence element of Comparative Example.

## Claims

1. A 1,3,5-tris(diarylamino)benzene represented by the general formula (I) 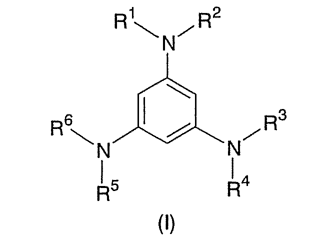 in which R¹ to R⁶ are each independently a group which is represented by the general formula (II) 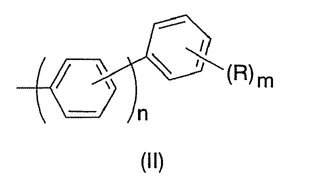 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that at least one of R¹ to R⁶ is independently a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

2. The 1,3,5-tris(diarylamino)benzene represented by the general formula (I) according to claim 1 in which at least one of R¹ to R⁶ is independently a group represented by the general formula (II) wherein m is 2, and n is 0, 1 or 2.

3. The 1,3,5-tris(diarylamino)benzene represented by the general formula (I) according to claim 1 in which at least one of R¹ to R⁶ is independently a group represented by the general formula (II) wherein m is 2, at least one of the two R's is situated at an ortho position, and n is 0, 1 or 2.

4. The 1,3,5-tris(diarylamino)benzene represented by the general formula (I) according to claim 1 in which at least one of R¹ to R⁶ is independently a group represented by the general formula (II) wherein m is 2, one of the two R's is situated at an ortho position and the other at the para position, and n is 0, 1 or 2.

5. A 1,3,5-tris(diarylamino)benzenes represented by the general formula (III) 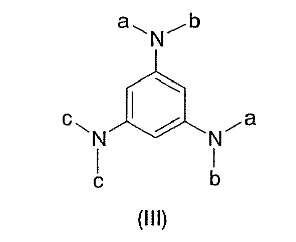 in which a, b and c are each independently a group represented by the general formula (II) 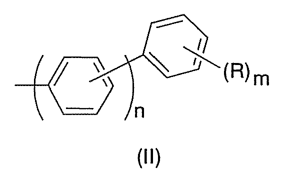 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

6. The 1,3,5-tris(diarylamino)benzene represented by the general formula (III) in which a is a group represented by the general formula (IIa) 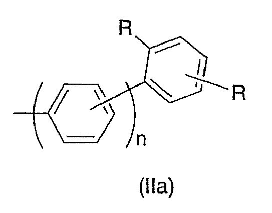 wherein the two R's are each independently an alkyl group of 1 to 6 carbon atoms or a cycloalkyl group of 5 or 6 carbon atoms, and n is 0, 1 or 2, and b and c are each independently a group represented by the general formula (IIb) 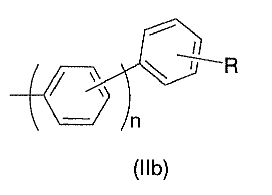 wherein R is independently a hydrogen atom, an alkyl group of 1 to 6 carbon atoms or a cycloalkyl group of 5 or 6 carbon atoms, and n is 0, 1 or 2.

7. A 1,3,5-tris(diarylamino)benzenes represented by the general formula (IV) 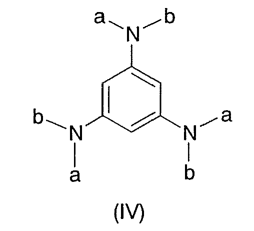 in which a and b are each independently a group represented by the general formula (II) 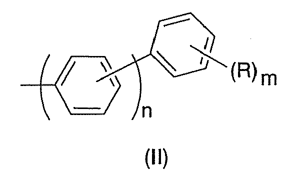 wherein R is independently a hydrogen atom, an alkyl group of 1 to 6 carbon atoms or a cycloalkyl group of 5 or 6 carbon atoms, m is 2, 3, 4 or 5, and n is 0, 1 or 2, provided that a is a group represented by the general formula (II) wherein R is an alkyl group of 1 to 6 carbon atoms or a cycloalkyl group of 5 or 6 carbon atoms, m is 2, 3, 4 or 5, and n is 0, 1 or 2.

8. The 1,3,5-tris(diarylamino)benzene represented by the general formula (IV) in which a is a group represented by the general formula (IIa) 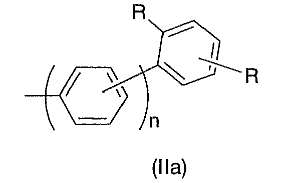 wherein the two R's are each independently an alkyl group of 1 to 6 carbon atoms or a cycloalkyl group of 5 or 6 carbon atoms, and n is 0, 1 or 2, and b is independently a group represented by the general formula (IIb) 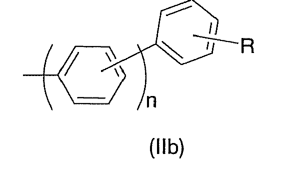 wherein R is independently a hydrogen atom, an alkyl group of 1 to 6 carbon atoms or a cycloalkyl group of 5 or 6 carbon atoms, and n is 0, 1 or 2.

9. N,N'-bis(2,4-dimethylphenyl)-N,N'-bis(4-terphenylyl)-N",N"-bis-(4-methylphenyl)-1,3,5-triaminobenzene.

10. N,N',N"-tris(2,4-dimethylphenyl)-N,N',N"-tris(4-terphenylyl)-1,3,5-triaminobenzene.

11. An organic electronic functional material comprising the 1,3,5-tris(diarylamino)benzene according to any one of claims 1 to 10.

12. An organic electroluminescence element comprising an organic electronic functional material according to claim 11.

13. An organic electroluminescence element comprising the organic electronic functional material according to claim 11 as a hole transporting and/or injecting agent.
